# EUROPEAN PATENT APPLICATION

(11) **EP 4 501 329 A1**
(43) Date of publication of application: **05.02.2025**
(21) Application number: 23780662.5
(22) Date of filing: 29.03.2023
(51) Int. Cl.: A61K 31/5513, A61K 9/08, A61P 27/06

(54) **STERILIZATION METHOD OF PHARMACEUTICAL PREPARATION AND PACKAGING**

(30) Priority: 30.03.2022 JP 2022056765
(71) Applicant: Santen Pharmaceutical Co., Ltd., Kita-ku Osaka-shi Osaka 530-8552 (JP)
(72) Inventor: ENDO, Yoko, Ikoma-shi, Nara 630-0101 (JP); MASAKI, Kenji, Ikoma-shi, Nara 630-0101 (JP); SEMBA, Kumi, Ikoma-shi, Nara 630-0101 (JP)
(74) Representative: Müller-Boré & Partner Patentanwälte PartG mbB
(86) International application number: PCT/JP2023/012751
(87) International publication number: WO 2023/190653

(57) **Abstract**

The present invention provides a pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a package which blocks a light having a wavelength of from 210 to 380 nm, in which the stability of (R)-11-[2-[2-[(diethylamino)methyl]-1-piperidinyl]acetyl]-5,11-dihydro-6H-pyrido[2,3-b][1,4]benzodiazepin-6-one or the salt thereof in the aqueous pharmaceutical composition is improved.

## Description

### TECHNICAL FIELD

The present invention relates to a pharmaceutical preparation.

### BACKGROUND ART

When a compound useful as an active pharmaceutical ingredient is developed as a medicament, it has been required to satisfy various requirements such as the stability of the compound in a pharmaceutical composition, the stability of a pharmaceutical composition itself, ensuring the preservative effect in the medicament besides the effectiveness and safety of the medicament. In order to produce the stable effect of medicaments, it is extremely important to ensure the stability of the compound in a pharmaceutical composition among them.

The compound useful as an active pharmaceutical ingredient may be unstable in a pharmaceutical composition's state such as aqueous solution even when the compound itself in a solid state is stable. In such case, this may cause problems such as the production of degradation products and the increase in the amount of the produced degradation products. For example, when a compound is reacted with water in an ingredient such as pharmaceutical additives and degraded, the compound may become unstable in a pharmaceutical composition. Also, the stability of a pharmaceutical composition may be affected by various physical factors such as pH, heat, humidity, light. Hence, it is also important that a pharmaceutical composition is stable against such various factors.

On the other hand, the stability of a compound in a pharmaceutical composition is often revealed as problems only after the pharmaceutical composition is prepared. Hence, no basic method of stabilizing a compound in a pharmaceutical composition is established and it is usual to find stabilization methods suitable for each compound.

It has been known that (R)-11-[2-[2-[(diethylamino)methyl]-1-piperidinyl]acetyl]-5,11-dihydro-6H-pyrido[2,3-b][1,4]benzodiazepin-6-one (hereinafter also referred to as "AFDX0250") is (+)-11-[2-[2-[(diethylamino)methyl]-1-piperidinyl]acetyl]-5,11-dihydro-6H-pyrido[2,3-b][1,4]benzodiazepin-6-one having the following structure and is useful for the treatment of an eye disease such as glaucoma, ocular hypertension and myopia (Patent Document 1 and Patent Document 2).

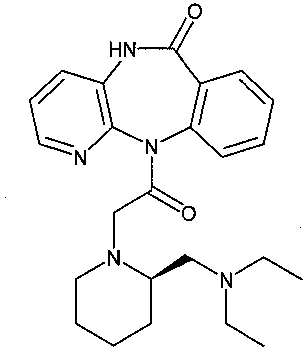

However, the stability of AFDX0250 or a salt thereof in an aqueous pharmaceutical composition has not been reported.

### PRIOR ART DOCUMENTS

### PATENT DOCUMENTS

Patent Document 1: WO 93/18772
Patent Document 2: WO 2022/030489

### SUMMARY OF THE INVENTION

### PROBLEMS TO BE SOLVED BY THE INVENTION

The present inventors have found that when AFDX0250 is dissolved to prepare an aqueous pharmaceutical composition, AFDX0250 is easily hydrolyzed and the hydrolysate of AFDX0250, 5,11-dihydro-6H-pyrido[2,3-b][1,4]benzodiazepin-6-one is easily precipitated at the stage of developing an aqueous pharmaceutical composition comprising AFDX0250 or a salt thereof. In addition, the present inventors have found that AFDX0250 or a salt thereof is easily degraded under light irradiation. The production of degradation products can be adequately inhibited in conventional preparations, but it would be very useful for the development of pharmaceutical preparations to further inhibit the production of such degradation products because such further inhibition thereof improves the quality of preparation compared to conventional preparations, leading to further improvements of the effectiveness and safety of preparations.

The object of the present invention is to provide a pharmaceutical composition with the improved stability (e.g., thermal stability and photostability) of AFDX0250 or a salt thereof in an aqueous pharmaceutical composition.

### SOLUTIONS TO THE PROBLEMS

The present inventors have studied to solve the object and have found that the storage of an aqueous pharmaceutical composition comprising AFDX0250 or a salt thereof in a package which blocks a light having a wavelength of from 210 to 380 nm (in particular, from 216 nm to 310 nm and 372 nm) can inhibit the degradation of AFDX0250 or a salt thereof under light exposure to ensure the stability of preparations. Also, the present inventors have found that the storage of an aqueous pharmaceutical composition comprising AFDX0250 or a salt thereof in a plastic container (in particular, polyethylene (PE) resin container, polypropylene (PP) resin container and polyethylene terephthalate (PET) resin container) inhibits the production of the hydrolysates of AFDX0250. In addition, the present inventors have found that a selected sterilization method (in particular, sterilization method by filtration using a filter) of an aqueous pharmaceutical composition comprising AFDX0250 or a salt thereof can effectively inhibit the production of the hydrolysates of AFDX0250 and AFDX0250-derived analogs in the aqueous pharmaceutical composition. Based on the findings, the present invention has been completed.

Specifically, the present invention provides the following embodiments.
[Item 1] A pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a package which blocks a light having a wavelength of from 210 to 380 nm
[Item 2] The pharmaceutical preparation according to the item 1, wherein the package is a package in which a transmittance of a light having a wavelength of from 210 to 380 nm or an average value thereof is 20% or less.
[Item 3] The pharmaceutical preparation according to the item 1, wherein the package is a package in which a transmittance of a light having a wavelength of from 210 to 380 nm or an average value thereof is 10% or less.
[Item 4] The pharmaceutical preparation according to any one of the items 1 to 3, wherein the package comprises a primary package and a secondary package.
[Item 5] The pharmaceutical preparation according to the item 4, wherein the primary package is a plastic container.
[Item 6] The pharmaceutical preparation according to the item 5, wherein the plastic container is a polyethylene resin container or a polyethylene terephthalate resin container.
[Item 7] The pharmaceutical preparation according to any one of the items 1 to 6, wherein the aqueous pharmaceutical composition is an aqueous pharmaceutical composition sterilized by filtration using a filter.
[Item 8] A pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a plastic container.
[Item 9] The pharmaceutical preparation according to the item 8, wherein the plastic container is a polyethylene resin container or a polyethylene terephthalate resin container.
[Item 10] The pharmaceutical preparation according to the item 8 or 9, wherein the aqueous pharmaceutical composition is an aqueous pharmaceutical composition sterilized by filtration using a filter.
[Item 11] A pharmaceutical preparation comprising an aqueous pharmaceutical composition sterilized by filtration using a filter comprising (R)-11-[2-[2-[(diethylamino)methyl]-1-piperidinyl]acetyl]-5,11-dihydro-6H-pyrido[2,3-b][1,4]benzodiazepin-6-one or a salt thereof.
[Item 12] The pharmaceutical preparation according to any one of the items 1 to 11 which is an eye drop.
[Item 13] A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises storing the aqueous pharmaceutical composition in a package which blocks a light having a wavelength of from 210 to 380 nm.
[Item 14] A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido [2,3-b][1,4]benzodiazepin-6-one or a salt thereof, wherein the method comprises storing the aqueous pharmaceutical composition in a plastic container.
[Item 15] A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises subjecting the aqueous pharmaceutical composition to sterilization by filtration using a filter.
[Item 16] A method of inhibiting the photolytic degradation of (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises storing an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof in a package which blocks a light having a wavelength of from 210 to 380 nm.
[Item 17] A method of enhancing the thermal stability of (R)-11-[2-[2-[(diethylamino)methyl]-1 -piperidinyl]acetyl]-5,11 -dihydro-6H-pyrido[2,3 - b][1,4]benzodiazepin-6-one or a salt thereof, wherein the method comprises storing an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof in a plastic container.
[Item 18] A method of inhibiting the production of 5,11-dihydro-6H-pyrido[2,3-b][1,4]benzodiazepin-6-one in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises storing the aqueous pharmaceutical composition in a plastic container.
[Item 19] A method of inhibiting the production of the analogs of (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises subjecting the aqueous pharmaceutical composition to sterilization by filtration using a filter.
Each feature of the above [Item 1] to [Item 19] may be optionally selected and combined two or more.

### EFFECTS OF THE INVENTION

According to the present invention, the stability such as thermal stability and photostability of AFDX0250 or a salt thereof in an aqueous pharmaceutical composition can be improved. Hence, in order to stabilize AFDX0250 or a salt thereof, it is not necessary to prepare a pharmaceutical composition comprising AFDX0250 or a salt thereof as a non-aqueous preparation.

In addition, the pharmaceutical preparation of the present invention can keep the stable amount of (R)-11-[2-[2-[(diethylamino)methyl]-1-piperidinyl]acetyl]-5,11-dihydro-6H-pyrido[2,3-b][1,4]benzodiazepin-6-one or a salt thereof. Hence, the pharmaceutical preparation has superior storage stability and safety and can be used and stored for a long period.

### BRIEF DESCRIPTION OF THE DRAWINGS

[Fig. 1] Fig. 1 shows absorption spectrum of AFDX0250 measured using ultraviolet visible spectrophotometer.
[Fig. 2] Fig. 2 shows the amounts (the residual ratios (%)) of AFDX0250 in test preparations (Preparations 1 and 2) at each irradiation wavelength after 24 hours of irradiation.
[Fig. 3] Fig. 3 shows the measurement results of transmittances of a light having each wavelength (%) for each type of containers and secondary packages packed in the containers.

### DETAILED DESCRIPTION

Hereinafter, the present invention is illustrated in detail.

The present invention provides a pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a package which blocks a light having a wavelength of from 210 to 380 nm.

In the present invention, the salt of (R)-11-[2-[2-[(diethylamino)methyl]-1-piperidinyl]acetyl]-5,11-dihydro-6H-pyrido[2,3-b][1,4]benzodiazepin-6-one is not particularly limited as long as it is a pharmaceutically acceptable salt. Examples of the salt include salts with an inorganic acid such as hydrochloric acid, hydrobromic acid, hydroiodic acid, nitric acid, sulfuric acid, phosphoric acid; salts with an organic acid such as acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid; salts with a metal such as sodium, potassium, calcium, magnesium; salts with an inorganic compound such as ammonia; and salts with an organic amine such as triethylamine, guanidine.

In the present invention, (R)-11-[2-[2-[(diethylamino)methyl]-1-piperidinyl]acetyl]-5,11-dihydro-6H-pyrido[2,3-b][1,4]benzodiazepin-6-one or a salt thereof contained in the pharmaceutical composition may be in the form of a hydrate or a solvate.

In the present invention, (R)-11-[2-[2-[(diethylamino)methyl]-1-piperidinyl]acetyl]-5,11-dihydro-6H-pyrido[2,3-b][1,4]benzodiazepin-6-one or a salt thereof may be prepared according to any well-known methods in the organic chemistry field or may be also obtained as a commercially available product. For example, (R)-11-[2-[2-[(diethylamino)methyl]-1-piperidinyl]acetyl]-5,11-dihydro-6H-pyrido[2,3-b][1,4]benzodiazepin-6-one can be prepared according to the step described in J. Med. Chem., 32(8), 1718-24, 1989.

In the present invention, the amount of (R)-11-[2-[2-[(diethylamino)methyl]-1-piperidinyl]acetyl]-5,11-dihydro-6H-pyrido[2,3-b][1,4]benzodiazepin-6-one or a salt thereof in the aqueous pharmaceutical composition is not particularly limited. The lower limit thereof is preferably 0.0001% (w/v), more preferably 0.0003% (w/v), furthermore preferably 0.0005% (w/v), even more preferably 0.001% (w/v), particularly preferably 0.0013% (w/v), particularly more preferably 0.0015% (w/v), particularly furthermore preferably 0.0017% (w/v), and most preferably 0.002% (w/v). The upper limit thereof is preferably 5% (w/v), more preferably 3% (w/v), furthermore preferably 2% (w/v), even more preferably 1% (w/v), particularly preferably 0.5% (w/v), particularly more preferably 0.2% (w/v), and most preferably 0.1% (w/v). From the viewpoint of preventing the precipitation of degradation products of (R)-11-[2-[2-[(diethylamino)methyl]-1-piperidinyl]acetyl]-5,11-dihydro-6H-pyrido[2,3-b][1,4]benzodiazepin-6-one or a salt thereof, the upper limit thereof is preferably 0.5% (w/v), more preferably 0.2% (w/v), and most preferably 0.1% (w/v). The amount of (R)-11-[2-[2-[(diethylamino)methyl]-1-piperidinyl]acetyl]-5,11-dihydro-6H-pyrido[2,3-b][1,4]benzodiazepin-6-one or a salt thereof is preferably 0.0001 to 5% (w/v), more preferably 0.0003 to 3% (w/v), furthermore preferably 0.0005 to 2% (w/v), even more preferably 0.001 to 1% (w/v), particularly preferably 0.0013 to 0.5% (w/v), particularly more preferably 0.0015 to 0.2% (w/v), particularly furthermore preferably 0.0017 to 0.1% (w/v), and most preferably 0.002 to 0.1% (w/v).

The term "% (w/v)" as used herein means the mass (g) of an object ingredient in 100 mL of the aqueous pharmaceutical composition of the present invention. When the aqueous pharmaceutical composition of the present invention comprise a salt of (R)-11-[2-[2-[(diethylamino)methyl]-1-piperidinyl]acetyl]-5,11-dihydro-6H-pyrido[2,3-b][1,4]benzodiazepin-6-one, the value may mean the amount of the salt of (R)-11-[2-[2-[(diethylamino)methyl]-1-piperidinyl]acetyl]-5,11-dihydro-6H-pyrido[2,3-b][1,4]benzodiazepin-6-one or the amount of (R)-11-[2-[2-[(diethylamino)methyl]-1-piperidinyl]acetyl]-5,11-dihydro-6H-pyrido[2,3-b][1,4]benzodiazepin-6-one. In addition, when the aqueous pharmaceutical composition of the present invention comprises (R)-11-[2-[2-[(diethylamino)methyl]-1-piperidinyl]acetyl]-5,11-dihydro-6H-pyrido[2,3-b][1,4]benzodiazepin-6-one or a salt thereof in the form of a hydrate or a solvate, the value may mean the amount (R)-11-[2-[2-[(diethylamino)methyl]-1-piperidinyl]acetyl]-5,11-dihydro-6H-pyrido[2,3-b][1,4]benzodiazepin-6-one or a salt thereof in the form of a hydrate or a solvate or the amount of (R)-11-[2-[2-[(diethylamino)methyl]-1-piperidinyl]acetyl]-5,11-dihydro-6H-pyrido[2,3-b][1,4]benzodiazepin-6-one or a salt thereof. Hereinafter, the same shall apply to the followings unless otherwise specified.

The term "aqueous pharmaceutical composition" as used herein means a pharmaceutical composition comprising water. The amount of water in the aqueous pharmaceutical composition of the present invention is not particularly limited, but the amount of water is preferably 50% (w/v) or more, more preferably 70% (w/v) or more, and furthermore preferably 80% (w/v) or more, relative to the aqueous pharmaceutical composition. In particular, the amount thereof is preferably 90% (w/v) or more, more preferably 95% (w/v) or more, and furthermore preferably 98% (w/v) or more, relative to the aqueous pharmaceutical composition.

In the present invention, the pH of the aqueous pharmaceutical composition is not particularly limited, but it is, for example, 6.5 or less, preferably 4 to 6.5, more preferably 5 to 6.3, furthermore preferably 5.3 to 6.2, and most preferably 5.5 to 6.

The aqueous pharmaceutical composition of the present invention may comprise a pharmaceutically acceptable active ingredient other than (R)-11-[2-[2-[(diethylamino)methyl]-1-piperidinyl]acetyl]-5,11-dihydro-6H-pyrido [2,3-b][1,4]benzodiazepin-6-one or a salt thereof, for example, an active ingredient with the effect of treating an eye disease such as glaucoma, ocular hypertension and myopia as long as the effect of the present invention is not prevented. Also, the aqueous pharmaceutical composition of the present invention may comprise no pharmaceutically acceptable active ingredient other than (R)-11-[2-[2-[(diethylamino)methyl]-1-piperidinyl]acetyl]-5,11-dihydro-6H-pyrido[2,3-b][1,4]benzodiazepin-6-one or a salt thereof. That is, the aqueous pharmaceutical composition of the present invention may comprise (R)-11-[2-[2-[(diethylamino)methyl]-1-piperidinyl]acetyl]-5,11-dihydro-6H-pyrido[2,3-b][1,4]benzodiazepin-6-one or a salt thereof as the sole active ingredient.

The aqueous pharmaceutical composition of the present invention may comprise an additive as appropriate. Examples of the additive include a buffering agent, a surfactant, a tonicity agent, a stabilizing agent, a preservative, an antioxidant, a high molecular weight polymer, a pH adjuster, and a base.

In the aqueous pharmaceutical composition of the present invention, a buffering agent available as pharmaceutical additives may be added as appropriate. Examples of the buffering agent include citric acid, acetic acid, malic acid, an amine, an amino acid, and a salt thereof. In the present invention, the buffering agent comprises citric acid or a salt thereof and is preferably used in combination with any of acetic acid or a salt thereof, malic acid or a salt thereof, an amine or a salt thereof, or an amino acid or a salt thereof.

Examples of the salt of citric acid include sodium citrate, disodium citrate, trisodium citrate, and others.

Examples of the salt of acetic acid include sodium acetate, potassium acetate, and others.

Malic acid may be in the form of a racemate or an enantiomer (L-body or D-body). Examples of the salt thereof include sodium malate, disodium malate, and others.

Examples of the amine include trometamol, monoethanolamine, diethanolamine, triethanolamine, meglumine, and others. Examples of the salt thereof include hydrochloride salt thereof, acetate salt thereof, and others. In the present invention, the amine is preferably trometamol and examples of the salt thereof include trometamol hydrochloride, and others.

Examples of the amino acid include ε-aminocaproic acid, glycine, alanine, valine, leucine, serine, threonine, methionine, glutamine, glutamic acid, asparagine, aspartic acid, arginine, lysine, histidine, and others. When they have optical isomers, they may be in the form of a racemate or an enantiomer (L-body or D-body). Examples of the salt thereof include hydrochloride salt thereof, acetate salt thereof, and others. In the present invention, the amino acid is preferably ε-aminocaproic acid, glycine, L-glutamine, and examples of the salt thereof include glycine hydrochloride, L-glutamine hydrochloride, and others.

In the present invention, the buffering agent may be in form of a hydrate or a solvate. Examples thereof include citric acid hydrate, sodium citrate hydrate, and others.

The lower limit of the amount of the buffering agent in the aqueous pharmaceutical composition of the present invention is preferably 0.001% (w/v), more preferably 0.005% (w/v), furthermore preferably 0.01% (w/v), even more preferably 0.05% (w/v), particularly preferably 0.1% (w/v), and most preferably 0.2% (w/v). The upper limit of the amount of the buffering agent in the aqueous pharmaceutical composition of the present invention is, for example, 20% (w/v), preferably 10% (w/v), more preferably 5% (w/v), furthermore preferably 4% (w/v), even more preferably 3% (w/v), particularly preferably 2% (w/v), and most preferably 1.5% (w/v). The amount of the buffering agent in the aqueous pharmaceutical composition of the present invention is, for example, 0.001 to 20% (w/v), preferably 0.001 to 10% (w/v), more preferably 0.005 to 5% (w/v), furthermore preferably 0.01 to 4% (w/v), even more preferably 0.05 to 3% (w/v), particularly preferably 0.1 to 2% (w/v), and most preferably 0.2 to 1.5% (w/v). For example, the amount of the buffering agent in the aqueous pharmaceutical composition of the present invention may be 0.001% (w/v), 0.002% (w/v), 0.005% (w/v), 0.01% (w/v), 0.02% (w/v), 0.05% (w/v), 0.1% (w/v), 0.2% (w/v), 1.5% (w/v), 2% (w/v), 3% (w/v), 4% (w/v), 5% (w/v), 6% (w/v), 8% (w/v), 10% (w/v), or 20% (w/v).

In an embodiment of the pharmaceutical preparation of the present invention, the aqueous pharmaceutical composition preferably comprises citric acid or a salt thereof and trometamol or a salt thereof as the buffering agent. In such case, the lower limits of the amounts of citric acid or a salt thereof and trometamol or a salt thereof in the aqueous pharmaceutical composition are preferably 0.0005% (w/v), more preferably 0.001% (w/v), furthermore preferably 0.005% (w/v), even more preferably 0.01% (w/v), particularly preferably 0.05% (w/v), and most preferably 0.1% (w/v), respectively. The upper limits thereof are preferably 10% (w/v), more preferably 5% (w/v), furthermore preferably 4% (w/v), even more preferably 3% (w/v), particularly preferably 2% (w/v), and most preferably 1.5% (w/v), respectively. The amounts of citric acid or a salt thereof and trometamol or a salt thereof in the aqueous pharmaceutical composition of the present invention are preferably 0.0005 to 10% (w/v), more preferably 0.001 to 5% (w/v), furthermore preferably 0.005 to 4% (w/v), even more preferably 0.01 to 3% (w/v), particularly preferably 0.05 to 2% (w/v), and most preferably 0.1 to 1.5% (w/v), respectively.

In the aqueous pharmaceutical composition of the present invention, a surfactant available as pharmaceutical additives such as a cationic surfactant, an anionic surfactant and a non-ionic surfactant may be added as appropriate.

Examples of the anionic surfactant include phosphorous lipid and others. Examples of the phosphorous lipid include lecithin and others.

Examples of the cationic surfactant include alkylamine salt, alkylamine-polyoxyethylene additive, fatty acid triethanolamine monoester salt, acylaminoethyldiethylamine salt, fatty acid-polyamine conjugate, trimethyl ammonium salt, dialkyl dimethyl ammonium salt, alkyl dimethyl benzyl ammonium salt, alkyl pyridinium salt, acylaminoalkyl-type ammonium salt, acrylaminoalkyl pyridinium salt, diacyloxyethyl ammonium salt, alkyl imidazoline, 1-acylaminoethyl 2-alkyl imidazoline, 1-hydroxylethyl-2-alkyl imidazoline, and others. Examples of the alkyl dimethyl benzyl ammonium salt include benzalkonium chloride, cetalkonium chloride, and others.

Examples of the non-ionic surfactant include polyethylene fatty acid ester, polyoxyethylene sorbitan fatty acid ester, polyoxyethylene hydrogenated castor oil, polyoxyethylene castor oil, polyoxyethylene polyoxypropylene glycol, sucrose fatty acid ester, vitamin E TPGS, and others.

Examples of the polyoxyethylene fatty acid ester include Polyoxyl 40 stearate and others.

Examples of the polyoxyethylene sorbitan fatty acid ester include Polysorbate 80, Polysorbate 65, Polysorbate 60, Polysorbate 40, polyoxyethylene sorbitan monolaurate, polyoxyethylene sorbitan trioleate, and others.

As the polyoxyethylene hydrogenated castor oil, various polyoxyethylene hydrogenated castor oils with different numbers of ethylene oxide polymerizations may be used, and the number of ethylene oxide polymerizations is preferably 10 to 100, more preferably 20 to 80, particularly preferably 40 to 70, and most preferably 60. Specific examples of the polyoxyethylene hydrogenated castor oil include polyoxyethylene hydrogenated castor oil 10, polyoxyethylene hydrogenated castor oil 40, polyoxyethylene hydrogenated castor oil 50, polyoxyethylene hydrogenated castor oil 60, and others.

As the polyoxyethylene castor oil, various polyoxyethylene castor oils with different numbers of ethylene oxide polymerizations may be used, and the number of ethylene oxide polymerizations is preferably 5 to 100, more preferably 20 to 50, particularly preferably 30 to 40, and most preferably 35. Specific examples of the polyoxyethylene castor oil include polyoxyl 5 castor oil, polyoxyl 9 castor oil, polyoxyl 15 castor oil, polyoxyl 35 castor oil, polyoxyl 40 castor oil, and others.

Examples of the polyoxyethylene polyoxypropylene glycol include polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, polyoxyethylene (20) polyoxypropylene (20) glycol, and others.

Examples of the sucrose fatty acid ester include sucrose stearate and others.

The vitamin E TPGS also means tocopherol polyethylene glycol 1000 succinate.

The amount of the surfactant in the aqueous pharmaceutical composition of the present invention may be suitably varied depending on, for example, the type of the surfactant. The amount thereof is preferably 0.001 to 10% (w/v), more preferably 0.01 to 5% (w/v), furthermore preferably 0.05 to 3% (w/v), and most preferably 0.1 to 2% (w/v).

In the aqueous pharmaceutical composition of the present invention, a tonicity agent available as pharmaceutical additives may be added as appropriate to adjust the osmotic pressure thereof. The osmotic pressure ratio of the aqueous pharmaceutical composition of the present invention is, for example, 1 and is not particularly limited as long as it is within pharmaceutically acceptable ranges. Examples thereof include an ionic tonicity agent, a non-ionic tonicity agent, and others.

Examples of the ionic tonicity agent include sodium chloride, potassium chloride, calcium chloride, magnesium chloride, and others. Examples of the non-ionic tonicity agent include glycerin, propylene glycol, sorbitol, mannitol, and others.

The amount of the tonicity agent in the aqueous pharmaceutical composition of the present invention may be suitably varied depending on, for example, the type of the tonicity agent. The amount thereof is preferably 0.01 to 10% (w/v), more preferably 0.02 to 7% (w/v), furthermore preferably 0.1 to 5% (w/v), particularly preferably 0.3 to 4% (w/v), and most preferably 0.5 to 3% (w/v).

In the aqueous pharmaceutical composition of the present invention, a stabilizing agent available as pharmaceutical additives may be added as appropriate. Examples of the stabilizing agent include edetic acid, sodium edetate, and others.

The amount of the stabilizing agent in the aqueous pharmaceutical composition of the present invention may be suitably varied depending on, for example, the type of the stabilizing agent. The amount thereof is preferably 0.001 to 10% (w/v), more preferably 0.01 to 5% (w/v), furthermore preferably 0.05 to 3% (w/v), and most preferably 0.1 to 2% (w/v).

In the aqueous pharmaceutical composition of the present invention, a preservative available as pharmaceutical additives may be added as appropriate. Examples of the preservative include benzalkonium chloride, benzalkonium bromide, benzethonium chloride, sorbic acid, potassium sorbate, methyl paraoxybenzoate, propyl paraoxybenzoate, chlorobutanol, and others.

The amount of the preservative in the aqueous pharmaceutical composition of the present invention may be suitably varied depending on, for example, the type of the preservative. The amount thereof is preferably 0.0001 to 1% (w/v), more preferably 0.0005 to 0.1% (w/v), furthermore preferably 0.001 to 0.05% (w/v), and most preferably 0.002 to 0.01% (w/v).

In the aqueous pharmaceutical composition of the present invention, an antioxidant available as pharmaceutical additives may be added as appropriate. Examples of the antioxidant include tocopherol, dibutylhydroxytoluene, butylhydroxyanisole, sodium erythorbate, propyl gallate, sodium sulfite, and others.

The amount of the antioxidant in the aqueous pharmaceutical composition of the present invention may be suitably varied depending on, for example, the type of the antioxidant. The amount thereof is preferably 0.0001 to 1% (w/v), more preferably 0.0005 to 0.1% (w/v), furthermore preferably 0.001 to 0.02% (w/v), and most preferably 0.005 to 0.010% (w/v).

In the aqueous pharmaceutical composition of the present invention, a high molecular weight polymer available as pharmaceutical additives may be added as appropriate. Examples of the high molecular weight polymer include methyl cellulose, ethyl cellulose, hydroxymethyl cellulose, hydroxyethyl cellulose, hydroxypropyl cellulose, hydroxyethyl methyl cellulose, hydroxypropyl methyl cellulose (hypromellose), carboxymethyl cellulose, sodium carboxymethyl cellulose, hydroxypropyl methyl cellulose acetate succinate, hydroxypropyl methyl cellulose phthalate, carboxymethyl ethyl cellulose, cellulose acetate phthalate, polyvinylpyrrolidone, polyvinyl alcohol, carboxyvinyl polymer, polyethylene glycol, and others. In the present invention, the high molecular weight polymer is preferably hydroxypropyl methyl cellulose (hypromellose).

The amount of the high molecular weight polymer in the aqueous pharmaceutical composition of the present invention may be suitably varied depending on, for example, the type of the high molecular weight polymer. The amount thereof is preferably 0.001 to 5% (w/v), more preferably 0.01 to 1% (w/v), and furthermore preferably 0.1 to 0.5% (w/v).

In the aqueous pharmaceutical composition of the present invention, a pH adjuster available as pharmaceutical additives may be added as appropriate as long as the pH thereof is 6.5 or less. Examples of the pH adjuster include hydrochloric acid, sodium hydroxide, potassium hydroxide, and others.

The amount of the pH adjuster in the aqueous pharmaceutical composition of the present invention may be suitably varied depending on, for example, the type of the pH adjuster. The amount thereof is preferably 0.001 to 5% (w/v), more preferably 0.01 to 1% (w/v), and furthermore preferably 0.1 to 0.5% (w/v).

In the aqueous pharmaceutical composition of the present invention, a base available as pharmaceutical additives may be added at appropriate. Examples of the base include water, purified water, saline, and others.

The aqueous pharmaceutical composition of the present invention may be administered orally or parenterally. Examples of the administration route include oral administration, intravenous administration, transdermal administration, and ocular topical administration (e.g., instillation administration, conjunctival sac administration, intravitreal administration, sub-conjunctival administration, sub-Tenon's administration), and others.

The pharmaceutical preparation of the present invention is not particularly limited as long as it is a dosage form that can be stored in packages described below. The pharmaceutical preparation may be used as a preparation for eye diseases, for example, a dosage form such as an eye drop, an eye gel, and an injection. The pharmaceutical preparation of the present invention is particularly preferably used as an eye drop.

They may be prepared according to any commonly-used methods in the art.

The pharmaceutical preparation of the present invention is provided by storing an aqueous pharmaceutical composition in a package which blocks a light having a specific wavelength range. The package may be capable of blocking a light having a specific wavelength range under the normal storage conditions expected for pharmaceutical preparations. The sealing property of the package is not particularly limited. Also, when an aqueous pharmaceutical composition is stored in the package, the aqueous pharmaceutical composition may be filled in usual manners depending on the characteristics of the package such as the form thereof.

The term "package" as used herein refers to a package for directly or indirectly storing the aqueous pharmaceutical composition of the present invention. Among the packages, the package for directly storing the aqueous pharmaceutical composition of the present invention refers to "primary package" and the package for indirectly storing the aqueous pharmaceutical composition of the present invention refers to "secondary package".

Examples of the primary package include a container for eye drop, a container for injection, a tube-shaped container, a bottle-shaped container, a spray container, and others. Examples of the secondary package include medicine envelope, aluminum pillow, box, shrink wrap, paper label, and others. Aluminum packages such as aluminum foil are not included within the scope of the primary and secondary packages.

In the present invention, the wavelength range of a light blocked by a package is from 210 to 380 nm, preferably from 215 to 340 nm and from 350 to 380 nm, more preferably from 216 to 310 nm and from 370 to 380 nm, and most preferably 216, 247, 278, 310 or 372 nm.

As used herein, the term "block(s) a light" having said wavelength range means that the transmittance of a light having the wavelength range is 50% or less or the light-shielding ratio is more than 50% or that the average value of transmittances of a light having the wavelength range is 50% or less or the average value of light-shielding ratios is more than 50%. For example, the "package which blocks a light having a wavelength of from 210 to 380 nm" refers to a package wherein the transmittance of a light having a wavelength of from 210 to 380 nm or the average value of the transmittances of a light having a wavelength of from 210 to 380 nm is 50% or less. From the viewpoint of further improving the stability to light, the transmittance of a light having a wavelength of from 210 to 380 nm or the average value thereof of the present invention is preferably 30% or less, more preferably 20% or less, furthermore preferably 15% or less, particularly preferably 10% or less, and most preferably 5% or less.

The method of measuring a transmittance of the present invention may be a method of measuring and calculating a transmittance of a light having a wavelength of from 210 to 800 nm using an ultraviolet visible spectrophotometer. It is not required that the entire package blocks a light having the wavelength range. Based on the inner surface of a package, the area which blocks a light having the wavelength range is preferably 50% or more, more preferably 60% or more, furthermore preferably 70% or more, even more preferably 80% or more, particularly preferably 90% or more, particularly more preferably 95% or more, and most preferably 100%, relative to the total area of the inner surface of the package. For example, an aspect in which a portion of the total area of the inner surface of a package blocks a light having the wavelength range is preferably a package in which a secondary package which blocks a light having the wavelength range (e.g., shrink, paper label) is wrapped around the side of a primary package which does not block a light having the wavelength range (e.g., container for eye drop).

When the pharmaceutical preparation of the present invention comprises a secondary package, a light having the above-described wavelength range may be blocked by any of a primary package or the secondary package. Also, even if a light having the above-described wavelength range is not blocked by each of the primary package and the secondary package alone, the light having the wavelength range can be blocked by a combination of the primary package and the secondary package. In such case, multiple secondary packages may be used. From the viewpoint that the pharmaceutical preparation of the present invention inhibits the degradation of AFDX0250 or a salt thereof not only during the distribution and storage thereof but also during use thereof, it is preferred that the primary package blocks a light having a specific wavelength range.

In the present invention, the material of a package is not particularly limited. Examples thereof include plastic, cellulose, pulp, rubber, metal, and others.

The primary package for directly storing the aqueous pharmaceutical composition of the present invention is preferably a plastic container, more preferably a polyethylene resin container, a polypropylene resin container and a polyethylene terephthalate resin container from the viewpoint of further stabilizing AFDX0250 or a salt thereof, and most preferably a polyethylene resin container and a polyethylene terephthalate resin container from the viewpoint of stabilizing AFDX0250 or a salt thereof for a longer period. Also, the primary package is preferably a transparent container in terms of internal visibility. When the primary package is a transparent container, the container may be transparent to the extent that the inner portion of the container can be visually checked. The term "transparent to the extent that the inner portion of the container can be visually checked" means that the transmittance of visible light (Wavelength: about 400 nm to about 800 nm) or the average value thereof is, for example, 20% or more, preferably 30% or more, more preferably 40% or more, furthermore preferably 50% or more, even more preferably 60% or more, particularly preferably 70% or more, particularly more preferably 80% or more, and most preferably 90% or more. It is not required that the entire container is transparent to the extent that the inner position of the container can be visually checked. Based on the outer surface of a package, the transparent area is preferably 30% or more, more preferably 50% or more, furthermore preferably 70% or more, even more preferably 80% or more, particularly preferably 90% or more, and most preferably 95% or more, relative to the total area of the outer surface of the package. The primary package may not be a transparent container.

The material of the secondary package for indirectly storing the aqueous pharmaceutical composition of the present invention is preferably plastic, cellulose, pulp, paper, and others from the viewpoint of the properties of the material such as processability. The secondary package may be not a transparent package. On the other hand, the secondary package may be a transparent package in terms of internal visibility. When the secondary package is a transparent package, the package may be transparent to the extent that the inner portion of the container can be visually checked. The term "transparent to the extent that the inner portion of the container can be visually checked" means that the transmittance of visible light (Wavelength: about 400 nm to about 800 nm) or the average value thereof is, for example, 20% or more, preferably 30% or more, more preferably 40% or more, furthermore preferably 50% or more, even more preferably 60% or more, particularly preferably 70% or more, particularly more preferably 80% or more, and most preferably 90% or more. It is not required that the entire secondary package is transparent to the extent that the inner position of the package can be visually checked. Based on the outer surface of a secondary package, the transparent area is preferably 1% or more, preferably 5% or more, more preferably 10% or more, furthermore preferably 30% or more, even more preferably 50% or more, particularly preferably 90% or more, and most preferably 95% or more, relative to the total area of the outer surface of the secondary package. Also, the secondary package may not be a transparent container or may have a transparent slit (visible window) for visually checking the inner position thereof.

In the present invention, the "polyethylene (PE) resin container" (hereinafter also referred to as "PE container") refers to a container in which the material of at least the portion in contact with an aqueous pharmaceutical composition of the container is polyethylene resin. Examples of the polyethylene include low-density polyethylene (LDPE), middle-density polyethylene (MDPE), high-density polyethylene (HDPE), and others. Also, the "polypropylene (PP) resin container" (hereinafter also referred to as "PP container") refers to a container in which the material of at least the portion in contact with an aqueous pharmaceutical composition of the container is polypropylene resin. The "polyethylene terephthalate (PET) resin container" (hereinafter also referred to as "PET container") refers to a container in which the material of at least the portion in contact with an aqueous pharmaceutical composition of the container is polypropylene terephthalate resin.

In addition, the pharmaceutical preparation preferably comprises a sterilization-treated aqueous pharmaceutical composition. Examples of the sterilization method of the aqueous pharmaceutical composition include electron beam (EB) sterilization, ethylene oxide gas (EOG) sterilization, hydrogen peroxide sterilization, gamma radiation sterilization, sterilization by filtration using a filter, autoclaved (AC) sterilization, and others. In the pharmaceutical preparation of the present invention, the sterilization method of the aqueous pharmaceutical composition is particularly preferably sterilization by filtration using a filter.

In the present invention, the "electron beam sterilization" or "EB sterilization" refers to a sterilization method capable of killing microorganisms such as bacteria using electron beam. The sterilization method is not particularly limited as long as it is a sterilization method of using electron beam. For example, the exposure dose may be appropriately selected from the range of 1 to 100 kGy and the exposure time may be appropriately selected from several seconds to several minutes.

In the present invention, the "ethylene oxide gas sterilization" or "EOG sterilization" refers to a sterilization method capable of killing microorganisms such as bacteria using ethylene oxide gas. The sterilization method is not particularly limited as long as it is a sterilization method of using ethylene oxide gas. For example, the sterilization temperature may be appropriately selected from 30 to 60 °C, the sterilization time may be appropriately selected from 1 to 10 hours, and the used gas may be ethylene oxide gas alone or the mixed gas of ethylene oxide gas and a gas such as carbon dioxide.

In the present invention, the "hydrogen peroxide sterilization" is a sterilization method capable of killing microorganisms such as bacteria using hydrogen peroxide. The sterilization method is not particularly limited as long as it is a sterilization method of using hydrogen peroxide.

In the present invention, the "gamma radiation sterilization" is a sterilization method capable of killing microorganisms such as bacteria using gamma radiation. The sterilization method is not particularly limited as long as it is a sterilization method of using gamma radiation.

In the present invention, the "sterilization by filtration using a filter " is a sterilization method of removing microorganisms such as bacteria via filtration using a membrane filter with a pore diameter of 0.22 µm. The membrane filter may be a filter made of a material such as PVDF (polyvinylidene fluoride), PES (polyethersulfone), PTFE (polytetrafluoroethylene) and MCE (mixed cellulose ester). Also, the membrane filter may be a filter with different pore diameter, for example, a pore diameter of 0.1 to 0.3 µm, a pore diameter of 0.2 to 0.25 µm, a pore diameter of 0.2 µm, a pore diameter of 0.21 µm, a pore diameter of 0.23 µm, a pore diameter of 0.24 µm, a pore diameter of 0.25 µm.

In the present invention, the "autoclaved (AC) sterilization" refers to a sterilization method capable of killing microorganisms such as bacteria using high-pressure steam sterilizer (autoclave). The sterilization method is not particularly limited as long as it is a sterilization method of using high-pressure steam sterilizer (autoclave). For example, the sterilization is performed under a condition, for example, at 115 to 118 °C for 30 minutes, at 121 to 124 °C for 15 to 20 minutes, or at 126 to 129 °C for 10 minutes and is preferably performed until the object product is in a sterile state.

When the pharmaceutical preparation of the present invention is used as an eye drop, the container for eye drop may be composed of one substance or may be composed of multiple substances. The aqueous pharmaceutical composition of the present invention may be stored in any of a one-piece container for eye drop composed of one substance, a two-piece container for eye drop composed of two substances or a three-piece container for eye drop composed of three substances. For example, the three-piece container for eye drop is composed of three substances of a container body for storing the aqueous pharmaceutical composition, an inner plug, and a cap. One-piece molded containers that are simultaneously blow-molded and filled with a drug solution also are included in the above container for eye drop based on the number of components of the containers. When containers are composed of multiple substances, they may be composed of substances made of the same materials or may be composed of components made of the different materials.

When the pharmaceutical preparation of the present invention is used as an eye drop, it is may be stored in any of a multi-dose container, a single-use unit-dose container or a PFMD (Preservative Free Multi Dose) container.

The pharmaceutical preparation of the present invention may be used for the treatment of an eye disease such as glaucoma, ocular hypertension, and myopia.

The present invention provides a pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a plastic container. The above description for the pharmaceutical preparation of the present invention is also applied to the pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a plastic container.

The aqueous pharmaceutical composition of the present invention is stored in a plastic container, preferably a polyethylene resin container, a polypropylene resin container or a polyethylene terephthalate resin container from the viewpoint of further stabilizing AFDX0250 or a salt thereof, and most preferably a polyethylene resin container or a polyethylene terephthalate resin container from the viewpoint of stabilizing AFDX0250 or a salt thereof for a longer period. The storage of the aqueous pharmaceutical composition in a plastic container particularly stabilizes (R)-11-[2-[2-[(diethylamino)methyl]-1-piperidinyl]acetyl]-5,11-dihydro-6H-pyrido[2,3-b][1,4]benzodiazepin-6-one or a salt thereof in the aqueous pharmaceutical composition compared to other containers.

The present invention provides a pharmaceutical preparation comprising an aqueous pharmaceutical composition sterilized by filtration using a filter comprising (R)-11-[2-[2-[(diethylamino)methyl]-1-piperidinyl]acetyl]-5,11-dihydro-6H-pyrido[2,3-b][1,4]benzodiazepin-6-one or a salt thereof. The above description for the pharmaceutical preparation of the present invention is also applied to the pharmaceutical preparation comprising an aqueous pharmaceutical composition sterilized by filtration using a filter comprising (R)-11-[2-[2-[(diethylamino)methyl]-1-piperidinyl]acetyl]-5,11-dihydro-6H-pyrido[2,3-b][1,4]benzodiazepin-6-one or a salt thereof.

The aqueous pharmaceutical composition of the present invention is an aqueous pharmaceutical composition sterilized by filtration using a filter. The sterilization method by filtration using a filter of an aqueous pharmaceutical composition particularly stabilizes (R)-11-[2-[2-[(diethylamino)methyl]-1-piperidinyl]acetyl]-5,11-dihydro-6H-pyrido[2,3-b][1,4]benzodiazepin-6-one or a salt thereof in the aqueous pharmaceutical composition compared to other sterilization methods.

The present invention provides a method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition. The above description for the pharmaceutical preparation of the present invention is also applied to the method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition.

The method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition of the present invention comprises a step of storing an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof in a package which blocks a light having a wavelength of from 210 to 380 nm. The method inhibits the degradation of (R)-11-[2-[2-[(diethylamino)methyl]-1-piperidinyl]acetyl]-5,11-dihydro-6H-pyrido[2,3-b][1,4]benzodiazepin-6-one or a salt thereof under light irradiation, and thus (R)-11-[2-[2-[(diethylamino)methyl]-1-piperidinyl]acetyl]-5,11-dihydro-6H-pyrido[2,3-b][1,4]benzodiazepin-6-one or a salt thereof becomes stable to light.

The method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition of the present invention comprises a step of storing an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof in a plastic container, preferably a step of storing the aqueous pharmaceutical composition in a polyethylene resin container, a polypropylene resin container or a polyethylene terephthalate resin container from the viewpoint of further stabilizing AFDX0250 or a salt thereof, and most preferably a step of storing the aqueous pharmaceutical composition in a polyethylene resin container or a polyethylene terephthalate resin container from the viewpoint of stabilizing AFDX0250 or a salt thereof for a longer period. The method inhibits the hydrolysis of (R)-11-[2-[2-[(diethylamino)methyl]-1-piperidinyl]acetyl]-5,11-dihydro-6H-pyrido[2,3-b][1,4]benzodiazepin-6-one, and thus can inhibit the production of the hydrolysate thereof, 5,11-dihydro-6H-pyrido[2,3-b][1,4]benzodiazepin-6-one. Also, (R)-11-[2-[2-[(diethylamino)methyl]-1-piperidinyl]acetyl]-5,11-dihydro-6H-pyrido[2,3-b][1,4]benzodiazepin-6-one or a salt thereof in the method becomes stable to heat.

The method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition of the present invention comprises a step of subjecting an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof to sterilization by filtration using a filter. The method can effectively inhibit the production of the hydrolysate thereof and (R)-11-[2-[2-[(diethylamino)methyl]-1-piperidinyl]acetyl]-5,11-dihydro-6H-pyrido[2,3-b][1,4]benzodiazepin-6-one derived analogs in the aqueous pharmaceutical composition.

### EXAMPLES

Hereinafter, the present invention is illustrated in Examples in order to make it easy to understand the present invention. The present invention, however, is not intended to be limited thereto by any means.

### Preparation Examples

Typical examples of preparations comprising the aqueous pharmaceutical composition of the present invention are shown below. The amount of each ingredient formulated in the following preparation examples (% (w/v)) is the amount (g) in 100 mL of the composition.

### [Preparation Example 1]

### Eye Drop (in 100 mL)

| | |
|---|---|
| AFDX0250 | 0.1 g |
| Sodium citrate hydrate | 0.5 g |
| Trometamol | 0.5 g |
| Glycerin | 2.0 g |
| Dilute hydrochloric acid | Appropriate quantity |
| Sodium hydroxide | Appropriate quantity |
| Purified water | Appropriate quantity |

Sterilization Method; Sterilization by filtration using a filter
Container; Polyethylene Resin Container (in which the transmittance of a light having a wavelength of from 210 to 380 nm is 10% or less)

### [Preparation Example 2]

### Eye Drop (in 100 mL)

| | |
|---|---|
| AFDX0250 | 0.3 g |
| Sodium citrate hydrate | 0.25 g |
| Citric acid hydrate | 0.02 g |
| ε-Aminocaproic acid | 0.5 g |
| Sodium chloride | 0.5 g |
| Dilute hydrochloric acid | Appropriate quantity |
| Sodium hydroxide | Appropriate quantity |
| Purified water | Appropriate quantity |

Sterilization Method; Sterilization by filtration using a filter
Container; Polyethylene Terephthalate Resin Container (in which the transmittance of a light having a wavelength of from 210 to 380 nm is 10% or less)

### [Preparation Example 3]

### Eye Drop (in 100 mL)

| | |
|---|---|
| AFDX0250 | 0.5 g |
| Sodium citrate hydrate | 0.25 g |
| Citric acid hydrate | 0.02 g |
| L-Malic acid | 0.5 g |
| Sodium chloride | 0.5 g |
| Dilute hydrochloric acid | Appropriate quantity |
| Sodium hydroxide | Appropriate quantity |
| Purified water | Appropriate quantity |

Sterilization Method; Sterilization by filtration using a filter
Container; Polyethylene Resin Container (in which the transmittance of a light having a wavelength of from 210 to 380 nm is 10% or less)

A desired composition may be prepared by appropriately adjusting the types and the amounts of AFDX0250, buffering agent and additive in said Preparation Examples 1 to 3.

### Example 1: Thermal Stability Test (1)

The thermal stability of AFDX0250 in a pharmaceutical preparation was evaluated by measuring the amount of the hydrolysate of AFDX0250, which is a byproduct, produced after the pharmaceutical Preparation was stored under a certain condition.

### (1) Test Preparation

The aqueous pharmaceutical composition with the composition shown in Table 1 (Preparation 1) was prepared in usual manners and then the prepared composition was filled in PE container, PP container, PET container and glass container to prepare each test preparation.

**[Table 1]**

| | Preparation 1 |
|---|---|
| AFDX0250 | 0.50% |
| Sodium chloride | 0.68% |
| Sodium hydroxide | Appropriate quantity |
| Dilute hydrochloric acid | Appropriate quantity |
| Water for injection | Appropriate quantity |
| pH | 6 |

### (2) Test Method

The prepared test preparations were stored at 25 °C for 1 month. The amount of 5,11-dihydro-6H-pyrido[2,3-b][1,4]benzodiazepin-6-one (hereinafter also referred to as "hydrolysate") produced by the hydrolysis of AFDX0250 in each test preparation after the 1 month storage was quantified by high performance liquid chromatography to calculate the production ratio of the hydrolysate (%). The production ratio of the hydrolysate (%) represents the ratio of the produced hydrolysate (%) when the amount of AFDX0250 in the test preparation is 100%.

### (3) Result

The production ratio of the hydrolysate of AFDX0250 in each test preparation (%) is shown in Table 2.

**[Table 2]**

| Material of Container | Preparation 1 |
|---|---|
| | Production ratio of hydrolysate (%) |
| PE | 0.58 |
| PP | 0.60 |
| PET | 0.60 |
| Glass | 0.98 |

As shown in the test results, the production ratio of hydrolysate was reduced when the aqueous pharmaceutical composition comprising AFDX0250 or a salt thereof was stored in the plastic containers compared to the glass container. Hence, it was shown that AFDX0250 had good stability in the aqueous pharmaceutical composition when AFDX0250 was stored in plastic containers.

### Example 2: Thermal Stability Test (2)

The thermal stability of AFDX0250 in a pharmaceutical preparation was evaluated by visually checking precipitates in the pharmaceutical preparation after the pharmaceutical preparation was stored under a certain condition.

### (1) Test Preparation

Preparation 1 prepared in Example 1 was filled in PE container, PP container, PET container and glass container to prepare each test preparation.

### (2) Test Method

The prepared test preparations were stored at 40 °C for 2 weeks. The presence or absence of the precipitates in each test preparation after the storage was visually checked.

### (3) Result

The test results are shown in Table 3. The "-" in the table means no generation of precipitates.

**[Table 3]**

| | Storage Period | Material of Container | | | |
|---|---|---|---|---|---|
| | | PE | PET | PP | Glass |
| Preparation 1 | 2 weeks | - | - | - | Precipitates |

As shown in the test results, it was confirmed that when the aqueous pharmaceutical composition comprising AFDX0250 or a salt thereof was stored in a glass container, precipitates were generated in the glass container after 2 weeks, whereas it was not confirmed that when the aqueous pharmaceutical composition was stored in plastic containers, no precipitate was generated in the containers. The results showed that AFDX0250 had good storage stability in the aqueous pharmaceutical composition when the aqueous pharmaceutical composition was stored in plastic containers.

### Example 3: Absorption Wavelength Measurement Test

The absorption wavelength of AFDX0250 was measured.

### (1) Test Preparation

AFDX0250 (10 mg) was dissolved in acetonitrile, and the solution was adjusted exactly to 100 mL to prepare 0.01% AFDX0250 solution. The solution was diluted 4-fold to prepare 0.0025% AFDX0250 solution. The prepared 0.0025% AFDX0250 solution was used as a test preparation.

### (2) Test Method

The absorption spectrum of the prepared test preparation was measured using ultraviolet visible spectrophotometer.

### (3) Result

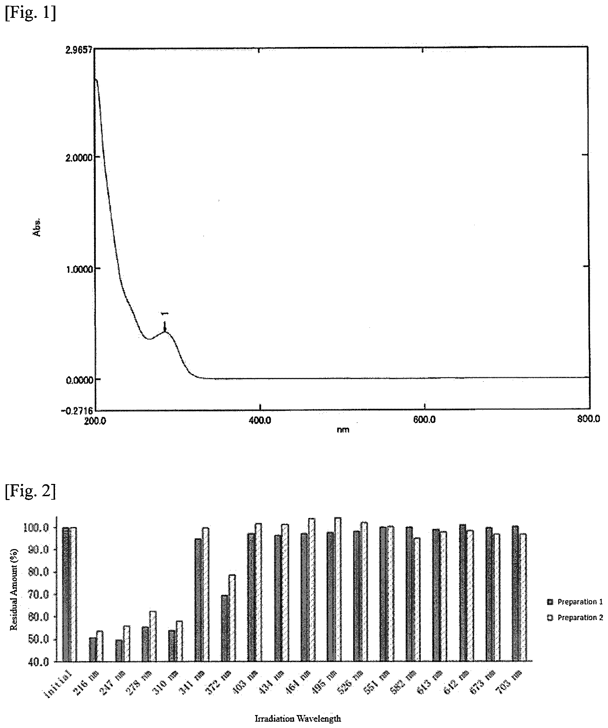

The test results are shown in Fig. 1.

As shown in the test results, no absorption of AFDX0250 was observed at the longer wavelengths than about 330 nm.

### Example 4: Photostability Test (1)

The stability of the aqueous pharmaceutical composition comprising AFDX0250 or a salt thereof under light irradiation was studied.

### (1) Test Preparation

Preparation 1 with the composition shown in the above Table 1 and Preparation 2 with the composition shown in Table 4 were prepared in usual manners. The prepared preparations were used as test preparations.

**[Table 4]**

| | Preparation 2 |
|---|---|
| AFDX0250 | 0.50% |
| Sodium citrate hydrate | 0.15% |
| Trometamol | 0.10% |
| Sodium chloride | 0.68% |
| Sodium hydroxide | Appropriate quantity |
| Dilute hydrochloric acid | Appropriate quantity |
| Water for injection | Appropriate quantity |
| pH | 6 |

### (2) Test Method

The prepared test preparations were irradiated at specific wavelengths using multi-wavelength irradiation spectrometer for 24 hours, and the amount of AFDX0250 in each test preparation at each irradiation wavelength after the irradiation was quantified by high performance liquid chromatography to calculate the residual ratio of AFDX0250 (%). The residual ratio of AFDX0250 (%) represents the ratio of AFDX0250 (%) after irradiation when the amount of AFDX0250 in the test formulation is 100%.

### (3) Result

The test results are shown in Fig. 2.

As shown in the test results, AFDX0250 was degraded at a wavelength of from 216 to 310 nm and 372 nm. It was observed that AFDX0250 was particularly degraded at a wavelength of 370 nm which is not absorption wavelength of AFDX0250. The results showed that the AFDX was unstable to a light having the limited specific wavelengths. That is, it was suggested that the photostability of AFDX0250 in the aqueous pharmaceutical composition could be ensured by blocking a light having the specific wavelength range including said wavelengths.

### Example 5: Photostability Test (2)

The stability of each pharmaceutical preparation in which the aqueous pharmaceutical composition comprising AFDX0250 or a salt thereof is stored in a package under light irradiation was studied.

### (1) Test Preparation

Preparation 2 prepared in Example 4 was filled in glass container, container for eye drop (A), container for eye drop (A) packed with shrink, container for eye drop (A) packed with paper label, container for eye drop (A) packed with box, containers for eye drop (A) packed with medicine envelope (A), medicine envelope (B), medicine envelope (C) or medicine envelope (D), container for eye drop (A) packed with aluminum pillow, container for eye drop (B), container for eye drop (C) and container for eye drop (D) to prepare each test preparation.

### (2) Test Method

The prepared test preparation was exposed to light at 1.2 million lux·hr or more, and then the amount of AFDX0250 in each test preparation was quantified by high performance liquid chromatography to calculate the residual ratio of AFDX0250 (%). The residual ratio of AFDX0250 (%) represents the ratio of AFDX0250 (%) after light exposure when the amount of AFDX0250 in the test preparation is 100%.

In addition, the transmittance of a light having each wavelength was measured for each type of containers and secondary packages packed in the containers.

### (3) Result

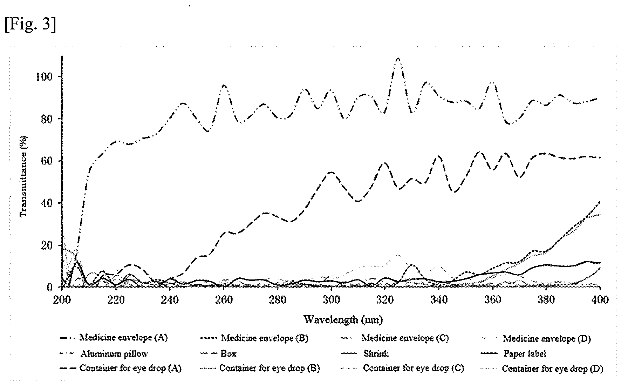

The residual ratios of AFDX0250 (%) for each package are shown in Table 5. In addition, the measurement results of transmittances of a light having each wavelength for each type of containers and secondary packages packed in the containers are shown in Fig. 3 and the wavelengths showing that the transmittances for each type of containers and secondary packages packed in the containers are 10% or less, 20% or less, 30% or less, and 50% or less are shown in Table 6. The transmittances of a light having a wavelength of 370 nm for each type of containers are shown in Table 7.

**[Table 5]**

| Packages | Preparation 2 |
|---|---|
| | Residual ratio of AFDX0250 (%) |
| Glass container | 93.5 |
| Container for eye drop (A) | 97.5 |
| Container for eye drop (A) + Shrink | 99.7 |
| Container for eye drop (A) + Paper label | 100.5 |
| Container for eye drop (A) + Box | 101.1 |
| Container for eye drop (A) + Medicine envelope (A) | 97.4 |
| Container for eye drop (A) + Medicine envelope (B) | 99.1 |
| Container for eye drop (A) + Medicine envelope (C) | 100.0 |
| Container for eye drop (A) + Medicine envelope (D) | 99.6 |
| Container for eye drop (A) + Aluminum pillow | 100.7 |
| Container for eye drop (B) | 99.8 |
| Container for eye drop (C) | 100.6 |
| Container for eye drop (D) | 100.2 |

**[Table 6]**

| Packages | Wavelength showing transmittance of 10% or less (nm) | Wavelength showing transmittance of 20% or less (nm) | Wavelength showing transmittance of 30% or less (nm) | Wavelength showing transmittance of 50% or less (nm) |
|---|---|---|---|---|
| Container for eye drop (A) | < 245 | < 255 | < 265 | < 300 |
| Container for eye drop (B) | < 365 | < 380 | < 395 | < 470 |
| Container for eye drop (C) | < 475 | < 490 | < 505 | < 745 |
| Container for eye drop (D) | < 740 | < 800 | < 800 | < 800 |
| Medicine envelope (A) | < 205 | < 205 | < 210 | < 210 |
| Medicine envelope (B) | < 365 | < 380 | < 395 | < 410 |
| Medicine envelope (C) | < 740 | < 800 | < 800 | < 800 |
| Medicine envelope (D) | < 520 | < 540 | < 565 | < 580 |
| Aluminum pillow | < 800 | < 800 | < 800 | < 800 |
| Box | < 800 | < 800 | < 800 | < 800 |
| Shrink | < 405 | < 405 | < 450 | < 795 |
| Paper label | < 380 | < 480 | < 705 | < 800 |

**[Table 7]**

| Packages | Transmittance of a light having a wavelength of 370 nm (%) |
|---|---|
| Container for eye drop (A) | 52.1 |
| Container for eye drop (B) | 11.2 |
| Container for eye drop (C) | 0.8 |
| Container for eye drop (D) | 2.9 |
| Medicine envelope (A) | 80.0 |
| Medicine envelope (B) | 11.9 |
| Medicine envelope (C) | 0.4 |
| Medicine envelope (D) | 5.0 |
| Aluminum pillow | 2.9 |
| Box | 0 |
| Shrink | 0 |
| Paper label | 5.7 |

As shown in the test results, it was confirmed that the photodegradation of AFDX0250 could be inhibited by storing the aqueous pharmaceutical composition comprising AFDX0250 or a salt thereof in the packages that reduce the transmittances of a light having a wavelength of from 216 to 310 nm and 372 nm to 50% or less. The results showed that the photostability of AFDX0250 in the aqueous pharmaceutical composition could be ensured by using the packages that reduce the transmittance of a light having the specific wavelength range including said wavelengths to 50% or less.

### Example 6: Stability Evaluation Test

An aqueous pharmaceutical composition comprising AFDX0250 or a salt thereof was sterilized to evaluate the stability of AFDX0250 in a preparation.

### (1) Test Preparation

Sodium citrate hydrate (2.5 g) was dissolved in purified water and the solution was adjusted exactly to 1 00 mL to prepare 2.5% sodium citrate hydrate solution. Citric acid hydrate (0.2 g) was dissolved in purified water and the solution was adjusted exactly to 100 mL to prepare 0.2% citric acid hydrate solution. Sodium chloride (5 g) was dissolved in purified water and the solution was adjusted exactly to 50 mL to prepare 10% sodium chloride solution. 0.25 g of AFDX0250 was weighed, the weighed AFDX0250 was mixed with 10 mL each of 2.5% sodium citrate hydrate solution, 0.2% citric acid hydrate solution and 10% sodium chloride solution, and then the solution was adjusted exactly to 50 mL with purified water to prepare a test preparation.

### (2) Test Method

The prepared test preparation was subjected to gamma radiation sterilization, sterilization by filtration using a filter or autoclaved (AC) sterilization to prepare a sterile formulation. The amount of AFDX0250 after each sterilization treatment was quantified by high performance liquid chromatography to calculate the residual ratio of AFDX0250 (%). The residual ratio of AFDX0250 (%) represents the ratio of AFDX0250 (%) after sterilization treatment when the amount of AFDX0250 in the test preparation is 100%. In addition, the amount of AFDX0250-derived hydrolysate (5,11-dihydro-6H-pyrido[2,3-b][1,4]benzodiazepin-6-one) in each test preparation excluding the test preparation subjected to gamma radiation sterilization was quantified by high performance liquid chromatography.

### (3) Result

The residual ratio of AFDX0250 (%) and the amount of the hydrolysate (%) in the test preparation after each sterilization treatment are shown in Table 8.

**[Table 8]**

| | Gamma radiation sterilization | Sterilization by filtration using a filter | AC sterilization |
|---|---|---|---|
| Residual ratio (%) | 55.7 | 100.3 | 93.8 |
| Hydrolysate (%) | - | 0.06 | 0.37 |

As shown in the test results, it was confirmed that the reduction in the residual ratio of AFDX0250 and the production of the hydrolysate of AFDX0250 were effectively inhibited in the preparation treated with the sterilization by filtration using a filter. The results showed that the pharmaceutical preparation of the present invention treated with the sterilization by filtration using a filter had better storage stability.

### INDUSTRIAL APPLICABILITY

According to the present invention, the stability such as thermal stability and photostability of AFDX0250 or a salt thereof in an aqueous pharmaceutical composition can be improved. Hence, the present invention can provide a pharmaceutical preparation with superior stability.

## Claims

1. A pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a package which blocks a light having a wavelength of from 210 to 380 nm.

2. The pharmaceutical preparation according to claim 1, wherein the package is a package in which a transmittance of a light having a wavelength of from 210 to 380 nm or an average value thereof is 20% or less.

3. The pharmaceutical preparation according to claim 1, wherein the package is a package in which a transmittance of a light having a wavelength of from 210 to 380 nm or an average value thereof is 10% or less.

4. The pharmaceutical preparation according to any one of claims 1 to 3, wherein the package comprises a primary package and a secondary package.

5. The pharmaceutical preparation according to claim 4, wherein the primary package is a plastic container.

6. The pharmaceutical preparation according to claim 5, wherein the plastic container is a polyethylene resin container, a polypropylene resin container, or a polyethylene terephthalate resin container.

7. The pharmaceutical preparation according to any one of claims 1 to 6, wherein the aqueous pharmaceutical composition is an aqueous pharmaceutical composition sterilized by filtration using a filter.

8. A pharmaceutical preparation comprising an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the aqueous pharmaceutical composition is stored in a plastic container.

9. The pharmaceutical preparation according to claim 8, wherein the plastic container is a polyethylene resin container, a polypropylene resin container, or a polyethylene terephthalate resin container.

10. The pharmaceutical preparation according to claim 8 or 9, wherein the aqueous pharmaceutical composition is an aqueous pharmaceutical composition sterilized by filtration using a filter.

11. A pharmaceutical preparation comprising an aqueous pharmaceutical composition sterilized by filtration using a filter comprising (R)-11-[2-[2-[(diethylamino)methyl]-1-piperidinyl]acetyl]-5,11-dihydro-6H-pyrido[2,3-b][1,4]benzodiazepin-6-one or a salt thereof.

12. The pharmaceutical preparation according to any one of claims 1 to 11 which is an eye drop.

13. A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises storing the aqueous pharmaceutical composition in a package which blocks a light having a wavelength of from 210 to 380 nm.

14. A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises storing the aqueous pharmaceutical composition in a plastic container.

15. A method of stabilizing (R)-11-[2-[2-[(diethylamino)methyl]-1-piperidinyl]acetyl]-5,11-dihydro-6H-pyrido[2,3-b][1,4]benzodiazepin-6-one or a salt thereof in an aqueous pharmaceutical composition comprising (R)-11-[2-[2-[(diethylamino)methyl]-1-piperidinyl]acetyl]-5,11-dihydro-6H-pyrido[2,3-b][1,4]benzodiazepin-6-one or a salt thereof, wherein the method comprises subjecting the aqueous pharmaceutical composition to sterilization by filtration using a filter.
